# Europäisches Patentamt

# European Patent Office

# Office européen des brevets

(19)

(11) Publication number: **0 005 569**

**B1**

(12) **EUROPEAN PATENT SPECIFICATION**

(45) Date of publication of patent specification: **04.11.81**

(51) Int. Cl.³: **B 01 J 31/16, C 07 B 1/00, C 07 B 29/00, C 07 C 1/04, C 07 C 27/22, C 07 C 45/28**

(21) Application number: **79200224.8**

(22) Date of filing: **07.05.79**

(54) Resin-ligand-transition metal complex composition and processes for using this composition as catalyst.

(30) Priority: **15.05.78 US 905813**

(43) Date of publication of application:
**28.11.79 Bulletin 79/24**

(45) Publication of the grant of the European patent:
**04.11.81 Bulletin 81/44**

(84) Designated Contracting States:
**BE DE FR GB IT NL**

(56) References cited:
**DE - A - 2 326 489**
**GB - A - 1 238 703**

(73) Proprietor: **SHELL INTERNATIONALE RESEARCH MAATSCHAPPIJ B.V.**
**Carel van Bylandtlaan 30**
**NL-2596 HR DEN HAAG (NL)**

(72) Inventor: **Kim, Leo**
**12126 Westmere Drive**
**Houston Texas 77077 (US)**
Inventor: **Paxson, Timm Edward**
**4403 Belle Hollow Drive**
**Houston Texas 77087 (US)**
Inventor: **Tang, Sunny Conrad**
**20231 Laurel Lock Drive**
**Katy, Texas 77450 (US)**

(74) Representative: **Keuzenkamp, Abraham et al,**
**c/o Shell Internationale Research Maatschappij B.V. P.O. Box 302**
**NL-2501 CH 's-Gravenhage (NL)**

Courier Press, Leamington Spa, England.

# 0 005 569

Resin-ligand-transition metal complex composition and processes for using
this composition as catalyst

The use of heterogeneous catalysts over homogeneous catalysts has several advantages such as allowing the use of fixed beds, ease of separation of catalyst from the product and catalyst recovery and regeneration.

Traditionally, to produce heterogeneous catalysts from metals of the transition element series, these metals have been deposited on inert supports such as alumina or silica. More recently metal catalysts have been covalently attached to inert resin backbones by use of diphenylphosphine or other ligands which are attached directly to the polymer and coordinately bound to the metal. Typical examples of this type are found in U.S. patent specification 3,998,864 and in Pittman et al, Chemtech, pages 560—566, 1973.

There has now been found a novel type of heterogeneous catalyst which is a complex composition comprising:

a) an ion exchange resin;

b) a transition metal which is directly bound either coordinately or ionically to the ion exchange resin, and

c) an organic linking compound which has at least one moiety co-ordinately bound to the metal and further has at least one moiety which is ionically bound to the ion exchange resin.

This heterogeneous catalyst is related to the heterogeneous catalyst of European Patent Application 782000358.6 (Publication No. 0002557), in which is disclosed a complex composition comprising an ion exchange resin with an organic linking compound ionically bound thereto and with the linking compound further co-ordinately bound to a transition element metal. The complex composition of this invention, on the other hand, not only has the transition element bonded to the linking compound but also to the resin. This dual bonding of the metal provides additional stability. The complex composition of this invention is much more leach-resistant with regard to the transition metal than conventional heterogeneous transition metal catalysts. The compositions of this invention are also relatively simple to prepare using commercially available compounds. Their preparations involve no exotic conditions and often times may be carried out in an aqueous solvent system and the resins may be easily stripped of metal and ligands for isolation of the metal species and regeneration of the catalyst. The resin-based catalysts of this invention have unique selectivity-reactivity properties when compared to their homogeneous analogues.

The ion-exchange resins utilized in the composition of this invention are well known in the art and are readily available commercially. These are in the gel form or are macroporous and are either strongly acidic, weakly acidic, strongly basic, intermediately basic, weakly basic or are of a mixed acid-base type. The strongly acidic resins typically are resins of cross-linked styrene, styrene/divinylbenzene, phenol formaldehyde, benzene formaldehyde, having functional sulphonic or phosphonic acid groups attached thereto. Also suitable are the fluorinated alkyl sulphonic acid resins containing the $-CFSO_3H$ group as, for example, the NAFION® type resins supplied by E.I. Du Pont de Nemours. The weakly acidic resins are those with carboxylic acid groups and are typically acrylic acid derivatives, such as, for example, those resins prepared by the copolymerization of methacrylic acid and divinyl benzene. Another weakly acidic resin is the chelating type which is a styrene-divinyl benzene copolymer containing iminocarboxylic acid such as iminodiacetic acid functional groups which can serve as an anion exchanger at very low pH. The basic resins typically are resins of cross-linked styrene, styrene-divinyl benzene, phenol-formaldehyde, benzene-formaldehyde, epoxy-polyamine, phenolic-polyamine having functional amine, either primary, secondary tertiary or quaternary, or pyridinium groups attached thereto. Typical examples of suitable commercially supplied resins are given in Table 1 of Bio-Rad Laboratories Catalogue, Chromatography, Electrophases, Immunochemistry and Membrane Filtration, Price List C, March, 1977.

The preferred resin choice for the composition of this invention will depend on the particular ionically bondable moiety utilized on the linking compound as well as on the particular use envisioned for the composition. For example, if the composition were used in a liquid-phase catalysis, the compositon and pH of the liquid would determine the preferred resin to be utilized.

The linking compound is hydrocarbyl, i.e., alkyl, aryl or mixtures of aryl and alkyl which alkyl can be either cyclic or acyclic or mixtures thereof, containing from 1 to 100 carbon atoms, preferably from 3 to 80 carbon atoms and has at least two moieties containing an atom other than carbon or hydrogen.

At least one moiety of the linking compound is in the ionic or ionizable form and is compatible with the exchange group on the ion-exchange resin, i.e., when the exchange group is acidic, the resin-compatible ionic moiety on the linking compound is basic-derived and vice versa. For basic-type resins and resin-compatible ion moiety is derived from carboxylic acid ($RCO_2^-$), phosphonic acid ($RPO(OH)O^-$), phosphinic acid ($R_2POO^-$), sulphenic acid ($RSO^-$), sulphinic acid ($RSOO^-$), sulphonic acid ($RSO_2O^-$), boronic acid ($RB(OH)O^-$), boronous acid ($RBO^-$). For acidic-type or mixed acid-base resins the resin-compatible ion moiety is mono-hydrocarbyl ammonium ($RN^+H_3$), dihydrocarbyl ammonium ($R_2N^+H_2$), trihydrocarbyl ammonium ($R_3N^+H$), quaternary ammonium ($R_4N^+$), pyridinium ($RC_5H_4N^+R_1$), phosphonium ($R_4P^+$), arsonium ($R_4As^+$) and sulphonium ($R_3S^+$).

2

The linking compound may have more than one of the ionic moieties. It may be polyfunctional, for example, in carboxylate ion, in phosphonate ion, in sulphonate ion, in quaternary ammonium ion or in pyridinium. The polyfunctional group may be the same or different.

At least one other moiety of the linking compound has an atom capable of complexing with transition metals, and consists of trivalent nitrogen, trivalent phosphorus, trivalent arsenic, trivalent bismuth and trivalent antimony.

The three valences of the complexing atoms may be satisfied by any organic radical: saturated or unsaturated aliphatic and/or saturated or unsaturated heterocyclic and/or aromatic radicals. These radicals may contain any functional group, such as carbonyl, nitro and hydroxy groups, as well as saturated and unsaturated alkyl groups and the radical may be bound to the complexing atom directly through a carbon-complexing atom linkage or through an electronegative atom, such as oxygen or sulphur.

It is also suitable for a simple organic radical to satisfy more than one of the valence of the complexing atom, thereby forming a heterocyclic compound with the trivalent complexing atom. For example, an alkylene radical may satisfy two of the valences thereby forming a cyclic compound. Another example would be the alkylene dioxy radical to form a cyclic compound where oxygen atoms link an alkylene radical to the complexing atom. In these two examples the third valence may be satisfied by any other organic radical.

The linking compound may have more than one of the metal-complexing moieties. It may be, for example, polydentate in phosphorus atom, e.g., it may be bi- or tridentate, having two or three phosphorus atoms. It may have mixed complexing atoms, e.g., a phosphorus and arsenic atom or two phosphorus atoms and one nitrogen atom, etc.

The trivalent nitrogen atom will be present as an amine, i.e., as a primary, secondary, tertiary, quaternary amine or as pyridine or cyanide. The trivalent phosphorus will be present as phosphine $(R_3P)$, phosphinite $(ROPR_2)$, phosphonite $(RO)_2PR$ and phosphite $(RO_3P)$. Correspondingly, trivalent arsenic will be available as arsine, arsinite, arsonite and arsenite; trivalent bismuth as bismuthine, bismuthinite, bismuthonite and bismuthite; and trivalent antimony as stibine, stibinite, stibonite and stibite. The preferred complexing atoms are phosphorus and nitrogen. The tertiary amines, phosphines, arsines and stibines and bismuthines have a marked tendency to form non-ionic complexes with metals.

When the linking compound is polydentate in an ionizable hetero-atom, it is understood that there will be a statistical distribution of the ionized atoms upon quaternization or protonation. For example, if one mole of a linking compound which contains 3 amine groups, is protonated with 2 miles of HCl, then some of the molecules of the linking compound will have 3 quaternized amine groups, some will have 2 and some will have 1, but on the average there will be 2 quaternized amino groups per molecule. It is further understood from general principles of organic chemistry that unit charges resulting from quaternization and protonation can be distributed as partial charges over several hetero-atoms in a linking compound molecule.

Thus, the linking compound as reacted in the composition of this invention will have at least one protonized or quaternized hetero-atom and at least one hetero-atom complexed with a transition metal. Suitable linking compounds utilized in making the composition of the invention include but are not limited to the following examples:

tris(dimethylamino)phosphine
tris(diethylamino)phosphine
tris(di-isopropylamino)phosphine
tris(methylethylamino)phosphine
tris(p-dimethylaminophenyl)phosphine
tris(p-diethylaminophenyl)phosphine
tris(p-methylethylaminophenyl)phosphine
tris(o-dimethylaminophenyl)phosphine
tris(m-dimethylaminophenyl)phosphine
tris(dimethylaminoethyl)phosphine
ethyl-bis(diphenylphosphinoethyl)amine.

Substitution of phosphinites, phosphonites, phosphites for the phosphine in the above compounds as well as arsines, arsinites, arsonites, arsenites, bismuthines, bismuthinites, bismuthonites, bismuthites, stibines, stibinites, stibonites, stibites and amines produces linking compounds useful in preparing the compositions of this invention. Other suitable compounds are:

2-(P,P-diphenylphosphino)benzoic acid
tris(beta-aminoethyl)amine
nicotinic acid
isonicotinic acid
2-chloronicotinic acid

3

1,1-dimethyl-4-phenyl piperazinium salt
2,2'-alkylarsino-1,1'-diphenylamine
2-(P,P-dicyclohexylphosphino)benzoic acid
beta-(dicyclohexylphosphino)propionic acid
1,4-(P,P-diphenylphosphino)benzene
2-diphenylphosphine-3-carboxy-2-butene
2-(P,P-diphenylphosphino)benzene sulphonic acid
2-amino-s-triazine
1-diphenylphosphino-2-diphenylphosphinoethane
bis-(diphenylphosphinoethyl)ethylamine
3-(dialkylphosphino)benzene phosphonic acid.

The preferred metals complexed with the linking compound are selected from the transition elements of the Periodic Table: Groups IVB, VB, VIB, VIIB, VIII, IB and IIB, technetium excluded. The metals are:

TABLE I

| IVB | VB | VIB | VIIB | VIII | | | IB | IIB |
|---|---|---|---|---|---|---|---|---|
| Ti | V | Cr | Mn | Fe | Co | Ni | Cu | Zn |
| Zr | Nb | Mo | — | Ru | Rh | Pd | Ag | Cd |
| Hf | Ta | W | Re | Os | Ir | Pt | Au | Hg |

More preferred metals are from Groups VIB, VIIB, VIII and IB.

The complexed metals can be in various oxidation states. See "Complexes of the Transition Metals with Phosphines, Arsines and Stibines" by G. Booth, Adv. Inorg. Nucl. Chem., 6 1—69 (1964) for a comprehensive description of complexes. For example, the Booth reference cites the following oxidation states for metals complexed with phosphines.

TABLE II

| Metal | Oxidation state for stable phosphine complexes |
|---|---|
| Ti | 4 |
| Zr | 4 |
| Hf | 4 |
| V | 0, 3, 4 |
| Cr | 0, 2, 3 |
| Mo | 0, 1, 2, 3, 4 |
| W | 0, 1, 2, 3, 4 |
| Mn | 0, 1 |
| Re | 0, 1, 2, 3, 4, 5 |
| Fe | 0, 1, 2, 3 |
| Ru | 0, 2, 3, 4 |
| Os | 2, 3, 4 |

**0 005 569**

TABLE II (Continued)

| Metal | Oxidation state for stable phosphine complexes |
|---|---|
| Co | 1, 2, 3 |
| Rh | 0, 1, 3 |
| Ir | 1, 3 |
| Ni | 0, 1, 2, 3 |
| Pd | 0, 2 |
| Pt | 0, 2 |
| Cu | 1, 3 |
| Ag | 1 |
| Au | 1, 3 |

Articles dealing with the complexing of amines with metals are "Inorganic Complexes", Jorgensen, C.K., Academic Press 1963, Chapter 4 and "Chemistry Coordination Compounds", Bailer (Ed.), Am. Chem. Soc. Monograph Series 131, 1956. The above references cite the following oxidation states for metals complexed with amines.

TABLE III

| Metal | Oxidation state for stable amine complexes |
|---|---|
| Cr | 0, 1, 2, 3 |
| Mo | 0, 3 |
| W | 2 and 3 (polynuclear) 4 (mononuclear) |
| Mn | 2 |
| Re | 3, 5 |
| Fe | 2, 3 |
| Ru | 2, 3 |
| Os | 2, 3, 4 |
| Co | 2, 3 |
| Rh | 3 |
| Ir | 3, 4 |
| Ni | 0, 2 |
| Pd | 2 |
| Pt | 0, 2, 4 |

### TABLE III (Continued)

| Metal | Oxidation state for stable amine complexes |
|-------|--------------------------------------------|
| Cu | 2 |
| Ag | 1, 2 |
| Au | 3 |

The metal is not only complexed with the linking compound but is also directly bound to the ion exchange resin. The metal will be typically ionically bound to the resin when the resin has acid functional groups attached thereto, such as sulphonic acid groups, phosphonic acid groups, fluorinated alkyl sulphonic acid groups and carboxylic acid groups. Coordinate covalent bonding occurs when the ion exchange resin has basic functional groups attached thereto, such as amino groups, either primary, secondary, tertiary or quaternary, or pyridinium groups or iminodiacetic acid groups.

The composition according to the invention may have more than one transition metal present. The composition may also have the metal(s) co-complexed with other ligands in addition to the linking compound. For example, from the above-noted Booth reference the metal-complexed moiety of the composition could have the following form and still be within the scope of the invention, i.e.:

$$M_Y \, M'_Z \, O_A \, H_B \, X_C \, (CN^-)_D (CO)_E (NO)_F (Cp)_G (P_y)_H (Acac)_I (AsR_3)_J$$
$$(NR_3)_K \, (PR_3)_L (SnX_3^-)_M (GeX_3^-)_M (Carb)_N P_Q (Funct.)_R.$$

$M_Y$ = metal in oxidation state shown in Table II or Table III
   $Y$ = zero is n mononuclear to polynuclear cluster

$M'_Z$ = metal in oxidation state shown before
   $Z$ = zero to n mononuclear of mixed metal polynuclear cluster where n is an integer greater than zero when $Y >$ zero and $Z >$ zero

$O$ = oxygen where
   $A$ = zero to n

$H$ = hydrogen where
   $B$ = zero to n

$X$ = halide, F, Cl, Br, I; where
   $C$ = zero to 5

$(CN^-)$ = cyanide where
   $D$ = zero to 5 when $y + z = 1$ or $D = 1$ to n when $y + z > 1$

$(CO)$ = carbonyl where
   $E$ = zero to 5 when $y + z = 1$ or $E = 1$ to n when $y + z > 1$

$(NO)$ = nitrosyl where
   $F$ = zero to 5 when $y + z = 1$ or $F = 1$ to n when $y + z > 1$

$Cp$ = cyclopentadienyl where
   $G$ = zero to 3 when $y + z = 1$ or $G = 1$ to n when $y + z > 1$

$Py$ = pyridine where
   $H$ = zero to 5 when $y + z = 1$ or $H = 1$ to n when $y + z > 1$

$Acac$ = acetylacetonate where
   $I$ = zero to 3 when $y + z = 1$ or $I = 1$ to n when $y + z > 1$

$(AsR_3)$ = arsines, where R = H, alkyl or aryl and
   $J$ = zero to 5 when $y + z = 1$ or $J = 1$ to n when $y + z > 1$
   the arsine also may be of the chelating type or contain mixed donating atoms, e.g.:

| | |
|---|---|
| $R_2 As$ | $R_2 As$ |
| $R'_2 N$ | $R'_2 P$ |

$(NR)_3$ = amines, where R = H, alkyl or aryl and
   $K$ = zero to 5 when $y + z = 1$ or $K = 1$ to n where $y + z > 1$ as with arsines, a chelating or mixed donor chelating ligand may be employed.

# 0 005 569

(PR$_3$)    = phosphines where R = H, alkyl, or aryl, and
L = zero to 5 when y+ z = 1 or L = 1 to n when y + z > 1 as with arsines, and amines, a chelating ligand may be employed.

(SnX$_3^-$) = or (GeX$_3^-$) = trihalostannyl or trihalogermyl where X = F, Cl, Br, I and M = zero to 5 when y + z = 1 or M = 1 to n when y + z > 1

(Carb.)  = carboxylate where N = zero to 5 when y + z = 1 or N = 1 to n when y + z > 1 P        = the bridging moiety/ligand between the metal and the resin support and Q = 1 to n.

(Funct.) = function ion exchange group attached to the ion exchange resin and R = 1 to n.

The compositions of the invention find use as catalysts in many chemical processes. Illustrative examples are the use of compositions containing rhodium or ruthenium complexes in hydroformylation of olefins with hydrogen and carbon monoxide, carbonylation of olefins with carbon monoxide and a hydrocrylic compound, hydrogenation, isomerization and Fischer-Tropsch reactions. Compositions containing cobalt complexes are useful in hydroformylation, carbonylation, hydrogenation and isomerization reactions. Compositions containing molybdenum complexes are used in disproportionation (metathesis) and isomerization reactions. Compositions containing palladium and platinum complexes are useful in hydroformylation, carbonylation, isomerization, hydrogenation and oligomerization/dimerization reactions. Compositions containing nickel complexes are useful in oligomerization/dimerization reactions. Compositions containing tungsten or rhenium complexes are useful in metathesis reactions.

The catalyst preparation procedures described in the Examples were carried out in nitrogen-filled dry boxes. The solvent benzene was purified by distillation over CaH$_2$, all other solvents were of reagent-grade and used as supplied. The phosphine [(CH$_3$)$_2$NC$_6$H$_4$]$_3$P, was used as supplied. The quaternized aminophosphines were prepared by reaction of one equivalent of CH$_3$Br with an aminophosphine in toluene solution at room temperature. The quaternized aminophosphine precipitated readily from the toluene solution. The resins are indicated by (resin backbone)-(exchange group), e.g., a sulphonated styrene-divinylbenzene resin would be (styrene-divinylbenzene)-(SO$_3^-$), etc. Ph, C$_6$H$_5$ and $\phi$— are used as abbreviations for phenyl; —$\phi$— and C$_6$H$_4$ indicated p-substituted benzene moieties.

## Example 1
### Preparation of carboxylated acrylic resin/RH III

A 10 g quantity of carboxylated acrylic resin Bio-Rex (registered Trade Mark) 70 (10.2 meq./g) was stirred with 1 litre of 1N NaCl at room temperature for 60 minutes. The solid was collected by filtration and the procedure repeated. The material was then washed thoroughly with de-ionized water and 23.4 ml of a 0.5% solution of Rh(NO$_3$)$_3$ in water. The combined materials were stirred overnight at room temperature. At the end of this time period, the material was collected by vacuum filtration and washed with de-ionized water until the washings were colourless. The material was air-dried.

## Example 2
### Preparation of carboxylated acrylic resin/RH III/methyl-quaternized [(CH$_3$)$_2$NC$_6$H$_4$]$_3$P compound

A 9.0 g lot of the material prepared in Example 1 was added to a solution of 2 litres of acetone-water (1:1 v/v) which contained 2.0 g (4.1 mmol.) of methyl-quaternized [(CH$_3$)$_2$NC$_6$H$_4$]$_3$P. These materials were stirred overnight at room temperature and the solids collected by vacuum filtration. The material was then washed with an acetone solution, a water solution, and finally air-dried. The material was analyzed to contain 0.10%w Rh.

## Example 3
### Preparation of phosphinated styrene-divinylbenzene resin/Rh III

A 10 g lot of Bio Rex (registered Trade Mark) 63 (microreticular gel. phosphinated, 6.6 meq./g) was treated as described in Example 1.

## Example 4
### Preparation of phosphinated styrene-divinylbenzene resin Rh III/methyl-quaternized [(CH$_3$)$_2$NC$_6$H$_4$]$_3$P compound

A 9.0 g lot of the material prepared in Example 3 was treated with the quaternized aminophosphine as described in Example 2. Rh analysis 0.45%w.

## Example 5
### Preparation of sulphonated styrene-divinylbenzene resin/Rh III

A 10 g lot of Rohm and Has XN1010Na (macroreticular resin, 3.6 meq./g) was treated in a manner similar to that described in Example 1.

## Example 6
### Preparation of sulphonated styrene-divinylbenzene resin/Rh III/quaternized [(CH$_3$)$_2$NC$_6$H$_4$]$_3$P compound

A 10.0 g lot of the material prepared in Example 5 was treated with the quaternized aminophosphine as described in Example 2. Rh analysis 0.45%w.

7

## Example 7

Preparation of sulphonated styrene-divinylbenzene resin/[(CH$_3$)$_2$NC$_6$H$_4$]$_3$P/platinum-tin cationic complex composition

The aminophosphine [(CH$_3$)$_2$NC$_6$H$_4$]$_3$P (14.0 g, 35.8 mmol.) was dissolved in 1000 ml warm benzene, cooled to room temperature, and filtered into a 2—1 round-bottomed flask quickly. 10.0 g of XN1010H$^+$ ion-exchange resin were added, and the mixture stirred magnetically on side of flask for 72 hours. The resin was then filtered, washed with benzene and vacuum-dried in an oven at 40°C. Analysis showed a resin/ligand material having the approximate formula (styrene-divinyl-benzene)-(SO$_3^-$)$_{1.5}$[([CH$_3$)$_2$NC$_6$H$_4$]$_3$P)(H$^+$)$_{1.5}$].

A CH$_2$Cl$_2$ solution of the platinum complex PtCl(CO).[P(C$_6$H$_5$)$_3$]$_2^+$ClO$_4^-$ was prepared by the addition of 0.30 g (1.4 mmol.) of anhydrous AgClO$_4$ to a solution of PtCl$_2$[P(C$_6$H$_5$)$_3$]$_2$ (1.05 g, 1.3 mmol.) dissolved in 40 ml of CO— saturated CH$_2$Cl$_2$. The CH$_2$Cl$_2$ solution was stirred under 3 bar of CO for $\frac{1}{2}$ hour, and filtered. To the resultant filtrate were added 5.0 g of the XN1010H$^+$ resin/ligand material, prepared as described above, mixed together on a rotator for approximately 70 hours and filtered. The resultant resin material was added to a solution of 5.0 g (22.2 mmol.) of SnCl$_2$.2H$_2$O dissolved in 450 ml of acetone, mixed on rotator for 1 hour, filtered, Soxhlet-extracted with refluxing benzene for 4 hours, and dried in vacuum oven overnight at approximately 40°C. Analysis showed a composition having the approximate formula (styrene-divinyl-benzene)-(SO$_3^-$)$_{1.5}$[(CH$_3$)$_2$NC$_6$H$_4$]$_3$-P(H$^+$)$_{1.5}$ [(PtCl(CO) [P(C$_6$H$_5$)$_3$]$_2$) (SnCl$_2$)$^+$]$_{0.07}$. The analytical results are shown in Table IV below.

TABLE IV

Analytical analysis of platinum-tin/phosphine/resin catalyst

|  | Neutron activation % by wt | Elemental % by wt | Analysis Relative Molar Value (carbon = 100) |
|---|---|---|---|
| C | — | 57.8 | 100 |
| H | — | 5.56 | 116 |
| S | — | 7.05 | 4.6 |
| N | — | 1.32 | 2.0 |
| P | 1.6 | 1.59 | 1.1 |
| Sn | 7.0 | 5.82 | 1.0 |
| Cl | — | 3.55 | 2.1 |
| Pt | 2.0 | 1.83 | 0.20 |

## Example 8

Preparation of sulphonated styrene-divinylbenzene resin/methyl-quaternised ([(CH$_3$)$_2$NC$_6$H$_4$]$_3$P)/ platinum-tin cationic complex composition. The quaternized aminophosphine ([(CH$_3$)$_2$NC$_6$H$_4$]$_3$P(CH$_3^+$)Br$^-$ (10.4 g (21.1 mmol.)) was dissolved in 1900 ml of an acetone/H$_2$O (17:7 v/v solution. 12.0 g of XN1010Na ion-exchange resin (prepared by exhaustive ion exchange of XN1010H$^+$ with 10 l of 1N NaCl or when the pH of the effluent wash was neutral) were added. The mixture was side-stirred for 48 hours, filtered with suction, and the resin washed with 5 × 100 de-ionized H$_2$O, then vacuum-dried in an oven overnight at 45°C. Analysis showed a resin/ligand material having the approximate formula (styrene-divinyl-benzene)-(SO$_3^-$)[([(CH$_3$)$_2$NC$_6$H$_4$]$_3$P)(CH$_3^+$)].

The material of this Example was prepared in a similar manner as in Example 7 except that the XN1010Na resin/ligand material prepared as described above was used. Analysis showed a composition having the approximate formula (styrene-divinyl-benzene)-(SO$_3^-$)[([(CH$_3$)$_2$NC$_6$H$_4$]$_3$P)(CH$_3^+$)] [(PtCl(CO)[P(C$_6$H$_5$)$_3$]$_2$)(SnCl$_2$)$^+$]$_{0.02}$.

*Process utilizing the compositions of this invention*

Example 9
Hydroformylation of hexene

To a 300-ml, ss Magnedrive autoclave (stirred at 600 r.p.m.) were added 70 ml of benzene, 2.0 ml of n-decane, 20.0 ml (160 mmol.) of 1-hexene, and 1.0 g of the catalyst as listed below. The solution was deoxygenated with nitrogen. Synthesis gas ($CO/H_2$, 1:1) was then charged to the reactor and the reactor was heated to the appropriate temperature listed below. Conversions and selectivities were obtained by gas chromatography; leach rates by atomic absorption. The leach rates are extremely low, in many cases undetectable. Results are given in Table V.

Hydroformylation with platinum/tin catalysts

| Catalyst | Temp., °C | Pressure, bar | Time, h | Conversion, % | Selectivities, % $C_7$-aldehyde hexane | | $C_7$-aldehyde linearity, % | Pt leach, ppm/h | Rate, m/m/h |
|---|---|---|---|---|---|---|---|---|---|
| of Example 7 | 80 | 216 | 10 | 16.4 | 96.5 | 3.5 | 94.3 | | |
| | | | 24 | 39.1 | 96.3 | 3.7 | 93.9 | | |
| | | | 44 | 52.7 | 95.5 | 4.5 | 93.2 | undetectatable | 170 |
| of Example 7 | 100 | 108 | 10 | 19.3 | 96.3 | 3.7 | 94.0 | | |
| | | | 24 | 35.6 | 94.5 | 5.2 | 93.1 | | |
| | | | 44 | 40.0 | 93.5 | 6.2 | 92.4 | undetectable | 170 |
| of Example 7 | 100 | 216 | 4 | 15.1 | 95.2 | 2.4 | 91.1 | | |
| | | | 10.5 | 36.1 | 97.2 | 2.1 | 88.8 | 0.03 | 180 |
| of Example 8 | 100 | 216 | 20 | 21.6 | 97.6 | 0.9 | 77.8 | undetectable | |
| a) | 80 | 216 | 10 | 3.4 | 95.5 | 4.5 | 90.4 | | |
| | | | 24 | 4.7 | 94.6 | 5.4 | 88.2 | | |
| | | | 45 | 2.4 | 93.7 | 6.3 | 81.6 | undetectable | 19 |

a) Homogeneous reaction, amount of catalyst $(PtCl_2(P\phi_3)_2, SnCl_2)$ identical to that in Example 7 above.

**0 005 569**

Example 10
Hydroformylation of 1,5-cyclo-octadiene (1,5—COD)

To a 300-ml, ss Magnedrive autoclave (stirred at 600 r.p.m.) were added 15 g (138 mmol.) of 1,5-cyclo-octadiene, 70 ml of tetrahydrofuran solvent, 2 g (10.1 mmol.) of n-tetra-decane and 0.5 g of catalyst material. The solution was deoxygenated with nitrogen. Synthesis gas (CO/H$_2$; 1:1 72 — 108 bar) was then charged to the reactor and the reactor was heated to 80—90°C. Conversions and selectivities were obtained by gas chromatography; leach rates by atomic absorption spectroscopy. Results are given in Table VI.

11

TABLE VI

Hydroformylation of 1,5-cyclo-octadiene

| Catalyst | Time, h | Total pressure bar | Conv., % | 1,3-COD | Selectivities, % | | | Total to $CHOC_8=$ | Leaching, ppm/h |
|---|---|---|---|---|---|---|---|---|---|
| | | | | | $4\text{-}CHOC_8=$ | $x\text{-}CHOC_8=$ | $CHOC_8\bullet$ | | |
| of Example 6 | 1.0 | 93 | 17.0 | 50.8 | 49.2 | 0 | 0 | 49.2 | |
| | 2.0 | | 34.2 | 48.7 | 51.3 | 0 | 0 | 51.3 | |
| | 3.0 | | 80.6 | 45.5 | 53.5 | 1.0 | trace | 54.5 | |
| | 4.0 | | 96.3 | 43.2 | 52.7 | 3.7 | 0.3 | 56.4 | |
| | 5.0 | | 99.1 | 45.6 | 48.2 | 6.2 | 0.3 | 54.4 | 2.5 |
| of Example 6 (first recycle) | 5.0 | 93 | 11.8 | 61.1 | 38.9 | 0 | 0 | 38.9 | |
| | 6.0 | | 25.7 | 65.3 | 34.7 | 0 | 0 | 34.7 | 0.17 |
| of Example 2 | 4.0 | 75 | 28.4 | 90.3 | 9.7 | 0 | 0 | 9.7 | 0.2 |
| of Example 4 | 4.0 | 72 | 18.6 | 85.2 | 14.5 | 0.4 | 0 | 14.9 | 1.0 |

$4\text{-}CHOC_8=$ :  4-formylcyclo-octene

$x\text{-}CHOC_8=$ :  the sum of all other isomeric formyloctenes

$CHOC_8\bullet$ :  saturated cyclo-octane aldehyde

**0 005 569**

### Claims

1. A complex composition comprising:

a) an ion exchange resin;

b) a transition metal which is directly bound either co-ordinately or ionically to the ion exchange resin, and

c) an organic linking compound which has at least one moiety co-ordinately bound to the metal and further has at least one moiety which is ionically bound to the ion exchange resin.

2. A composition as claimed in claim 1, characterized in that the ion exchange resin is a strongly acidic, weakly acidic or mixed acid-base type and the linking compound contains from 1 to about 100 carbon atoms, the ionically bound moiety of the linking compound is selected from the group consisting of monohydrocarbyl ammonium, dihydrocarbyl ammonium, trihydrocarbyl ammonium, quaternary ammonium, pyridinium, phosphonium, arsonium and sulphonium ion and the co-ordinately bound moiety contains a hetero-atom selected from the group consisting of trivalent nitrogen, trivalent phosphorus, trivalent arsenic, trivalent bismuth and trivalent antimony.

3. A composition as claimed in claim 2, characterized in that the functional group of the ion exchange resin is derived from sulphonic acid, fluorinated alkyl sulphonic acid, phosphonic acid, carboxylic acid or iminocarboxylic acid.

4. A composition as claimed in claim 3, characterized in that the ion exchange resin has a backbone of polystyrene, polystyrene cross-linked with divinyl benzene, phenol-formaldehyde condensate, benzene-formaldehyde condensate, polyacrylic acid or polymethacrylic acid.

5. A composition as claimed in claim 1, characterised in that the ion exchange resin is a basic-type resin, the linking compound contains from 1 to about 100 carbon atoms, the ionically bound moiety of the linking compound is derived from the group consisting of carboxylic acid, phosphonic acid, phosphinic acid, sulphenic acid, sulphinic acid, sulphonic acid, boronic acid and boronous acid and the co-ordinately bound moiety contains a hetero-atom selected from the group consisting of trivalent nitrogen, trivalent phosphorus, trivalent arsenic, trivalent bismuth and trivalent antimony.

6. A composition as claimed in claim 5, characterized in that the functional group of the ion exchange resin is selected from the group consisting of primary, secondary, tertiary, quaternary amine and pyridinium.

7. A composition as claimed in claim 6, characterized in that the ion exchange resin has a backbone of polystyrene, polystyrene cross-linked with divinyl benzene, phenol-formaldehyde condensate, benzene-formaldehyde condensate, epoxy polyamine and phenolic polyamine.

8. A composition as claimed in claims 1—7, characterized in that the transition metal is from Group VIB, VIIB, VIII or IB.

9. A process of hydroformylating olefins with hydrogen and carbon monoxide, characterized in that is used as a catalyst a composition as claimed in claims 1—8, wherein the transition metal is cobalt, ruthenium, palladium, platinum or rhodium.

10. A process for carbonylating olefins with carbon monoxide and a hydroxylic compound, characterized in that is used as a catalyst a composition as claimed in claims 1—8, wherein the transition metal is cobalt, ruthenium, palladium, platinum or rhodium.

11. A process for hydrogenating an organic compound, characterized in that is used as a catalyst a composition as claimed in claims 1—8, wherein the transition metal is cobalt, ruthenium, platinum, palladium or rhodium.

12. A process of isomerizing organic compounds, characterized in that is used as a catalyst a composition as claimed in claims 1—8, wherein the transition metal is cobalt, molybdenum, ruthenium, palladium, platinum or rhodium.

13. A process of disproportionating olefins, characterized in that is used as a catalyst a composition as claimed in claims 1—8, wherein the transition metal is molybdenum, tungsten or rhenium.

14. A process of oligomerizing organic compounds, characterized in that is used as a catalyst a composition as claimed in claims 1—8, wherein the metal is nickel, palladium or platinum.

15. A process of preparing hydrocarbons by reacting hydrogen and carbon monoxide, characterized in that is used as a catalyst a composition as claimed in claims 1—8, wherein the metal is ruthenium or rhodium.

### Revendications

1. Composition complexe comprenant:

a) Une résine échangeuse d'ions

b) un métal de transition qui est lié directement par liaison coordonnée ou ionique à la résine échangeuse d'ions et

c) un composé organique d'enchaînement qui a au moins une portion liée par liaison coordonnée au métal et de plus a au moins une portion qui est liée ioniquement à la résine échangeuse d'ions.

2. Composition selon la revendication 1, caractérisée en ce que la résine échangeuse d'ions est

13

une résine fortement acide, faiblement acide ou de type mixte acide-base et la composé d'enchaînement contient de 1 à environ 100 atomes de carbone, la portion liée ioniquement du composé d'enchaînement est choisie parmi des ions monohydrocarbyl ammonium, dihydrocarbylammonium, tri-hydrocarbylammonium, ammonium quaternaire, pyridinium, phosphonium, arsonium et sulfonium et la portion liée par liaison coordonnée contient un hétéro-atome choise parmi ceux d'azote trivalent, de phosphore trivalent, d'arsenic trivalent, de bismuth trivalent et d'antimoine trivalent.

3. Composition selon la revendication 2, caractérisée en e que le groupe fonctionnel de la résine échangeuse d'ions est dérivé d'acide sulfonique, d'acide alcoylsulfonique fluoré, d'acide phosphonique, d'acide carboxylique ou d'acide imino-carboxylique.

4. Composition selon la revendication 3, caractérisée en ce que la résine échangeuse d'ions a une squelette de polystyrène, de polystyrène réticulé par du divinylbenzène, de produit de condensation phénol-formaldéhyde, de produit de condensation benzène-formaldéhyde, d'acide polyacrylique ou d'acide polyméthacrylique.

5. Composition selon la revendication 1, caractérisée en ce que la résine échangeuse d'ions est une résine de type basique, le composé d'enchaînement contient de 1 à environ 100 atomes de carbone, la portion ioniquement liée du composé d'enchaînement est dérivée d'acide carboxylique, d'acide sulphosphonique, d'acide phosphinique, d'acide sulfénique, d'acide sulfinique, d'acide sulfonique, d'acide boronique ou d'acide boroneux et la portion liée par liaison coordonnée contient un hétéro-atome choisi parmi ceux d'azote trivalent, de phosphore trivalent, d'arsenic trivalent, de bismuth trivalent et d'antimoine trivalent.

6. Composition selon la revendication 5, caractérisée en ce que le groupe fonctionnel de la résine échangeuse d'ions est choisi parmi les groupes amine primaires, secondaires, tertiaires, quaternaires et le groupe pyridinium.

7. Composition selon la revendication 6, caractérisée en ce que la résine échangeuse d'ions a un squelette de polystyrène, de polystyrène réticulé par du divinylbenzène, de produit de condensation phénol-formaldéhyde, de produit de condensation benzène-formaldéhyde, d'epoxy-polyamine ou de polyamine phénolique.

8. Composition selon l'une quelconque des revendications 1 à 7 caractérisée en ce que le métal de transition est choisi dans les groupes VIB, VIIB, VIII ou IB,

9. Procédé pour l'hydroformylation d'oléfines avec de l'hydrogène et de l'oxyde de carbone, caractérisé en ce qu'on utilise comme catalyseur une composition telle que revendiquée dans l'une des revendications 1 à 8, dans laquelle le métal de transition est du cobalt, du ruthénium, du palladium, du platine ou du rhodium.

10. Procédé pour la carbonylation d'oléfines avec de l'oxyde de carbone et un composé hydroxylé, caractérisé en ce qu'on utilise comme catalyseur une composition telle que revendiquée dans l'une des revendications 1 à 8, dans laquelle le métal de transition est du cobalt, du ruthénium, du palladium, du platine ou du rhodium.

11. Procédé pour hydrogéner un composé organique, caractérisé en ce qu'on utilise comme catalyseur une composition telle que revendiquée dans l'une des revendications 1 à 8, dans laquelle le métal de transition est du cobalt, du ruthénium, du platine, du palladium ou du rhodium.

12. Procédé pour isomériser des composés organiques, caractérisé en ce qu'on utilise comme catalyseur une composition telle que revendiquée dans l'une des revendications 1 à 8, dans laquelle le métal de transition est du cobalt, du molybdène, du ruthénium, du palladium, du platine ou du rhodium.

13. Procédé pour la dismutation d'oléfines, caractérisé en ce qu'on utilise comme catalyseur une composition telle que revendiquée dans l'une des revendications 1 à 8, dans laquelle le métal de transition est du molybdène, du tungstène ou du rhénium.

14. Procédé d'oligomerisation de composés organiques, caractérisé en ce qu'on utilise comme catalyseur une composition telle que revendiquée dans l'une des revendications 1 à 8, dans laquelle le métal est du nickel, du palladium ou du platine.

15. Procédé de préparation d'hydrocarbures en faisant réagir de l'hydrogène et de l'oxyde de carbone, caractérisé en ce qu'on utilise comme catalyseur une composition telle que revendiquée dans l'une des revendications 1 à 8, dans laquelle le métal est du ruthénium ou du rhodium.


**Patentansprüche**

1. Komplex-Zusammensetzung, enthaltend
a) ein Ionenaustauscherharz;
b) ein Übergangsmetall, das direkt, entweder koordinativ oder ionisch, an das Ionenaustauscher-harz gebunden ist und
c) eine organische Brückenverbindung, die zumindest einen koordinativ an das Metall gebundenen Molekülteil sowie zumindest einen ionisch an das Ionenaustauscherharz gebundenen Molekülteil aufweist.

2. Zusammensetzung nach Anspruch 1, dadurch gekennzeichnet, dass das Ionenaustauscherharz vom stark sauren, schwach sauren oder gemischt sauer-basischen Typ ist und dass die Brückenverbindung 1 bis etwa 100 Kohlenstoffatome enthält, der ionisch gebundene Molekülteil der Brückenverbindung aus der Gruppe ausgewählt ist, die ein mit 1, 2 oder 3 Kohlenwasserstoffresten substituiertes Ammonium-, quaternäres Ammonium-, Pyridinium-, Phosphonium-, Arsonium- oder Sulphoniumion umfasst, und dass der koordinativ gebundene Molekülteil ein Heteroatom enthält, ausgewählt aus der Gruppe, die dreiwertigen Stickstoff, dreiwertigen Schwefel, dreiwertiges Arsen, dreiwertiges Wismut und dreiwertiges Antimon umfasst.

3. Zusammensetzung nach Anspruch 2, dadurch gekennzeichnet, dass die funktionelle Gruppe des Ionenaustauscherharzes von Sulfonsäure, fluorierter Alkylsulfonsäure, Phosphonsäure, einer Carbonsäure oder einer Iminocarbonsäure abgeleitet ist.

4. Zusammensetzung nach Anspruch 3, dadurch gekennzeichnet, dass das Ionenaustauscherharz ein Grundskelett aus Polystyrol, mit Divinylbenzol vernetztem Polystyrol, Phenolformaldehyd-Kondensationsprodukt, Benzolformaldehyd-Kondensationsprodukt, Polyacrylsäure oder Polymethacrylsäure aufweist.

5. Zusammensetzung nach Anspruch 1, dadurch gekennzeichnet, dass das Ionenaustauscherharz ein basisches Harz ist, die Brückenverbindung 1 bis etwa 100 Kohlenstoffatome enthält, der ionisch gebundene Molekülteil der Brückenverbindung aus der Gruppe abgeleitet ist, die Carbonsäure, Phosphonsäure, Phosphinsäure, Sulfensäure, Sulfinsäure, Sulfonsäure, Boronsäure und boronige Säure umfasst, und dass der koordinativ gebundene Molekülteil ein Heteroatom aus der Gruppe ist, die dreiwertigen Stickstoff, dreiwertigen Phosphor, dreiwertiges Arsen, dreiwertiges Wismut und dreiwertiges Antimon umfaßt.

6. Zusammensetzung nach Anspruch 5, dadurch gekennzeichnet, dass die funktionelle Gruppe des Ionenaustauscherharzes aus der Gruppe ausgewählt ist, die primäres, sekundäres, tertiäres sowie quaternäres Amin und Pyridium umfasst.

7. Zusammensetzung nach Anspruch 6, dadurch gekennzeichnet, dass das Ionenaustauscherharz ein Grundskelett aus Polystyrol, mit Divinylbenzol vernetztem Polystyrol, Phenolformaldehyd-Kondensationsprodukt, Benzolformaldehyd-Kondensationsprodukt, Epoxypolyamin oder phenolischem Polyamin aufweist.

8. Zusammensetzung nach den Ansprüchen 1 bis 7, dadurch gekennzeichnet, dass das Übergangsmetall der Gruppe VIB, VIIB, VIII oder IB, angehört.

9. Verfahren zur Hydroformylierung von Olefinen mit Wasserstoff und Kohlenmonoxid, dadurch gekennzeichnet, dass als Katalysator eine Zusammensetzung nach den Ansprüchen 1 bis 8 verwendet wird, in der das Übergangsmetall Kobalt, Ruthenium, Palladium, Platin oder Rhodium ist.

10. Verfahren zur Carbonylierung von Olefinen mit Kohlenmonoxid und einer Hydroxyverbindung, dadurch gekennzeichnet, dass als Katalysator eine Zusammensetzung nach den Ansprüchen 1 bis 8 verwendet wird, in der das Übergangsmetal Kobalt, Ruthenium, Palladium, Platin oder Rhodium ist.

11. Verfahren zur Hydrierung einer organischen Verbindung, dadurch gekennzeichnet, dass als Katalysator eine Zusammensetzung nach den Ansprüchen 1 bis 8 verwendet wird, in der das Übergangsmetall Kobalt, Ruthenium, Platin, Palladium oder Rhodium ist.

12. Verfahren zur Isomerisierung organischer Verbindungen, dadurch gekennzeichnet, dass als Katalysator eine Zusammensetzung nach den Ansprüchen 1 bis 8 verwendet wird, in der das Übergangsmetall Kobalt, Molybdän, Ruthenium, Palladium, Platin oder Rhodium ist.

13. Verfahren zur Disproportionierung von Olefinen, dadurch gekennzeichnet, dass als Katalysator eine Zusammensetzung nach den Ansprüchen 1 bis 8 verwendet wird, in der das Übergangsmetall Molybdän, Wolfram oder Rhenium ist.

14. Verfahren zur Oligomerisierung organischer Verbindungen, dadurch gekennzeichnet, dass als Katalysator eine Zusammensetzung nach den Ansprüchen 1 bis 8 verwendet wird, in der das Metall Nickel, Palladium oder Platin ist.

15. Verfahren zur Herstellung von Kohlenwasserstoffen durch Umsetzung von Wasserstoff und Kohlenmonoxid, dadurch gekennzeichnet, dass als Katalysator eine Zusammensetzung nach den Ansprüchen 1 bis 8 verwendet wird, in der das Metall Ruthenium oder Rhodium ist.